Europäisches Patentamt

European Patent Office

Office européen des brevets

(11) Publication number: **0 013 049**
**B1**

## EUROPEAN PATENT SPECIFICATION

(45) Date of publication of patent specification: **13.04.83**

(21) Application number: **79200763.5**

(22) Date of filing: **14.12.79**

(51) Int. Cl.³: **C 10 K 1/12,** B 01 D 53/14,
B 01 D 53/34, C 01 B 3/52,
C 07 C 7/11, C 10 K 1/14

(54) Process for acid gas removal.

(30) Priority: **28.12.78 GB 5012178**

(43) Date of publication of application:
**09.07.80 Bulletin 80/14**

(45) Publication of the grant of the patent:
**13.04.83 Bulletin 83/15**

(84) Designated Contracting States:
**BE DE FR GB NL**

(56) References cited:
**DE - A - 2 531 898**
**DE - B - 1 273 115**
**FR - A - 2 228 719**

(73) Proprietor: **SHELL INTERNATIONALE RESEARCH MAATSCHAPPIJ B.V.**
**Carel van Bylandtlaan 30**
**NL-2596 HR  Den Haag (NL)**

(72) Inventor: **Van de Kraats, Eduard Johan**
**Badhuisweg 3**
**1031 CM Amsterdam (NL)**
Inventor: **Darton, Richard Charles**
**Carel van Bylandtlaan 30**
**2596 HR The Hague (NL)**

(74) Representative: **Puister, Antonius Tonnis, Mr. et al,**
**P.O. Box 302**
**NL-2501 CH  The Hague (NL)**

Process for acid gas removal

The invention relates to a process for the removal of acid gases from mixtures containing them by contacting the said mixtures in an absorber with a solvent comprising a chemical absorbent and between 5 and 55% by weight of water, regenerating the loaded solvent thus obtained by heating it in a regenerator so that at least part of the water present in the solvent boils and the greater part of the absorbed acid gases is released, recirulating the regenerated solvent to the absorber, withdrawing the resulting water vapor and acid gases from the regenerator, recondensing water vapour from the withdrawn gases, returning condensate thus obtained to the upper part of the regenerator and adding back condensate to the regenerated solvent after it has left the regeneration zone of the regenerator.

In a process of the above type, as described in French patent application 2,228,719 and German Auslegeschrift 1,273,115, the acid gas-containing mixture is contacted with the solvent which preferentially absorbs the acid gases. The solvent which has thus absorbed acid gases is referred to as "fat solvent". A treated feed having a decreased acid gas content leaves the top of the absorber. The fat solvent is regenerated in the regenerator by stripping the absorbed acid gases from it using steam. This steam may be injected as such into the lower part of the regenerator and/or it may be formed in situ from the water present in the fat solvent by heating the regenerator at its lower end by means of a reboiler. The regenerated solvent (also referred to as "lean solvent") is then recirculated to the absorber and the released acid gases are removed for further treatment.

The invention provides a process for the removal of acid gases from mixtures containing them by contacting the said mixtures in an absorber with a solvent comprising a chemical absorbent and between 5 and 55% by weight of water, regenerating the loaded solvent thus obtained by heating it in a regenerator so that at least part of the water present in the solvent boils and the greater part of the absorbed acid gases is released, recirculating the regenerated solvent to the absorber, withdrawing the resulting water vapour and acid gases from the regenerator, recondensing water vapour from the withdrawn gases, returning condensate thus obtained to the upper part of the regenerator and adding back condensate to the regenerated solvent after it has left the regeneration zone of the regenerator, which process is characterized in that condensate is removed from a tray in the regenerator above the regeneration zone.

According to the invention the condensate, before being added back to the regenerated solvent, is partly or wholly returned to the upper part of the regenerator where it is used to wash the rising gas to remove traces of the solvent from it, and condensate is removed from the regenerator above the regeneration zone by suitable means, such as a so-called total draw-off tray. This removal of condensate results in the additional advantages of reducing the loss of solvent from the regenerator and of much better washing of the gases leaving the regenerator.

In the context of this specification $H_2S$, $CO_2$ and COS are considered as acid gases. These acid gases are often to be removed from mixtures containing them. The said mixtures may be in the liquid state, such as gasoline, or they may be in the gaseous state, such as natural gas, propane, butane and mixtures thereof.

A chemical absorbent is a compound which reacts with acid gases; in general, such an absorbent has basic properties. Very suitably the chemical absorbent consists of one or more basic amines and may comprise a primary, secondary and/or tertiary amine. Alkanolamines are suitable, especially those having 1 to 4 and preferably 2 to 3 carbon atoms per alkanol radical. Typical examples are monoethanolamine, diethanolamine, diisopropanolamine, diethylethanolamine, methyldiethanolamine and mixtures thereof. Other amines which can be used are alkylamines, phenylalkylamines, alkoxyalkyl- and alkoxyarylamines. Typical of these are phenylethylamine, and methoxydiethyldiamine. Aliphatic polyamines having between 2 and 8 amine groups per molecule may also be used. Examples of such amines are triethylenetetramine, tetraethylenepentamine and derivatives thereof.

The solvent very conveniently also comprises a physical absorbent for acid gases. The acid gases do not react with a physical absorbent, but are able to dissolve therein. Physical absorbents must be soluble in the water-containing solution to be contacted with the acid gas-containing mixtures.

The physical absorbent preferably comprises at least one component selected from cyclotetramethylenesulphones, aliphatic acid amides, N-alkylated pyrrolidones, N-alkylated piperidones, glycol ethers, ether-ketones or their derivatives.

Derivatives for cyclotetramethylenesulphone, which is also known as sulfolane, should have not more than 4, more preferably not more than 2 alkyl substituents in the tetramethylenesulphone ring. Sulfolane is the preferred species of this class of compounds.

Suitable aliphatic acid amides are the N-dialkyl substituted aliphatic acid amides, a preferred species being N,N-dimethylformamide. The alkyl groups directly attached to the nitrogen atom should have from 1 to 4 carbon atoms each while an acid with 1 to 4 carbon atoms per molecule is preferred. Apart from N,N-dimethylformamide mentioned above other species in this sub-class include N-methyl-N-ethylformamide, N,N-diethylformamide, N-propyl-N-methylformamide, N,N-dibutylformamide, N,N-dimethylacetamide, N-methyl-N-ethylacetamide, formamide and acetamide.

Where an N-alkylated pyrrolidone or an N-alkylated piperidone is used, although the alkyl substituent on the nitrogen atom may be any alkyl group, alkyl groups with 1 to 4 carbon atoms are preferred and N-methylpyrrolidone is particularly suitable.

Very suitable glycol ethers are dialkyl ethers of polyalkylene glycols, such as the dimethyl and diethyl ether of polyethylene glycols.

Examples of ether-ketones which are very suitable to be used as physical absorbents are ethoxy- and propoxyketones such as 2-methoxy-2-methyl-3-butanone and 2-methoxy-2-methyl-4-pentanone.

Preferably the solvent comprises between 10 and 35% by weight of water.

The term "regeneration zone" is used here to mean that zone in the regenerator in which the solvent is contacted with rising vapour, and excluding any reboiling zone at the tottom of the regenerator or exterior to it.

The reduced mass of water in the regeneration zone, which has to be reboiled continuously, improves thermodynamic conditions for stripping and changes the chemical equilibrium in the solvent.

Reducing the water content means that, at the same temperature the solvent has a lower total vapour pressure. Because of the lower total pressure the volume of the gas stream is relatively greater for a given mass flow of steam. This will cause the partial pressure of the acid component to be further from its equilibrium value and hence the driving force for mass transfer (stripping) to be greater. However it will usually not be possible to operate the regenerator at lower pressure since this is determined by downstream units. In this case the pressure may be kept the same, and providing there are no heat transfer constraints, the temperature may be raised. This again is advantageous for the stripping since at a higher temperature the acid gas is less soluble.

In addition, the reduced proportion of water in the solvent reduces the strength of the chemical bond between the chemical absorbent in the solvent and the acid gas, which lowers the resistance of the absorbed gases to stripping.

A further saving can be made in capital costs due to the reduced size of the regenerator since the flows of both vapour and liquid in the regeneration zone are reduced.

To make a significant effect on the energy consumption required for regeneration of a solvent, the solvent should not contain so much water that its removal from the regeneration zone does not substantially alter the thermodynamic acid chemical conditions obtaining there. Some water/steam should, nevertheless, remain in the regenerator as this is necessary for the removal of the $H_2S$ from the regenerator. Furthermore, sufficient water should be present in the solvent that a significant proportion of it can be withdrawn from the regenerator without the temperature at the bottom of the regenerator becoming unstable due to excessively low partial pressure of the remaining water; a remedy for this phenomenon is proposed below.

The optimum conditions obtain when the solvent comprises a chemical and a physical absorbent and contains between 15 and 25% by weight of water. Then up to about two thirds, but more usually between one third and a half of the water may by-pass the regeneration zone so that the effect is most marked and a reduction of stripping steam consumption of some 20% to 25% may be obtainable, though more usually it will be in the order of 15%.

Some of the recondensed water may be recirculated to the upper part of the regenerator as a water wash in order to prevent or at least reduce the loss of the solvent from the regenerator by being carried downstream to the acid gas treatment plant. Alternatively, the condensate may be used to wash the acid gas in a small vessel external to the regenerator.

In order to maintain the water inventory in the solvent the condensate removed from the regenerator above the regeneration zone is added back to it after, i.e. downstream of, the regeneration zone, conveniently after the solvent has left the regenerator. Depending upon the temperature of the condensate it will be added to the regenerated solvent before or after the latter has been cooled by heat exchange with the fat solvent and/or in a cooler.

According to another embodiment of the invention at least a part of the condensate is added back to the solvent in the reboiler or at the bottom of the regeneration vessel, in other words after the regeneration zone. This can considerably simplify the control of the operation of the regenerator where the solvent is highly concentrated and little water is present. In this case, the low proportion of water in the solvent can result in a loss of temperature stability because of the excessive influence of the water vapour partial pressure on the total pressure in the regenerator. By adding at least a part of the condensate back into the reboiler, it increases the bulk of the water thus reducing the concentration of the absorbents in the solvent in the reboiler without affecting the improved thermodynamic conditions in the regeneration zone. The extra heat heeded to reheat the increased bulk of water to the regeneration operating temperature is small compared with the benefit obtained.

In a very attractive embodiment of the invention the condensate is added back to part of the regenerated solvent and the mixture thus obtained is introduced at the top of the absorber. In this way a very good removal of $H_2S$ from the feed is achieved. The remainder of the regenerated solvent is introduced into the absorber at a lower point.

Very suitably the absorption process is carried out at a temperature between 30°C and 80°C, in particular between 40°C and 60°C and at super atmospheric pressure, whereas the regeneration step is carried out at a temperature from 100°C to 200°C, e.g. 140°C, at a somewhat elevated pressure e.g. of $1\frac{1}{2}$ to 2 bar.

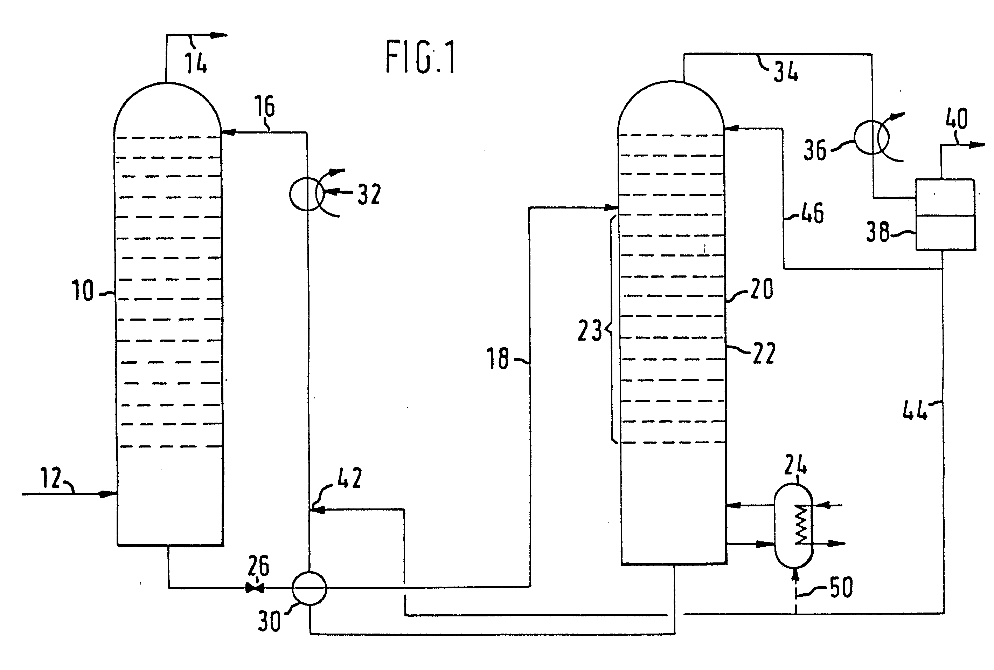

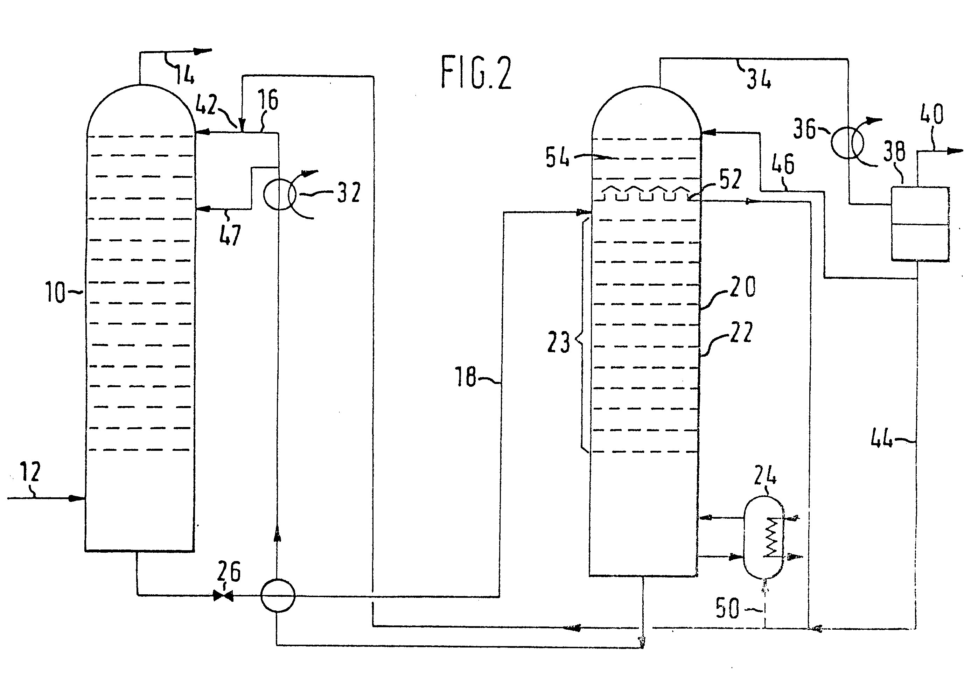

The invention will now be further described by way of example with reference to the accompanying drawings in which: Figure 1 is a schematic block diagram of an acid gas absorption plant and Figure 2 shows a plant for carrying out a process according to the invention.

A hydrocarbon feed containing acid gas enters the lower end of an absorber 10 by a line 12.

As shown, the absorber 10 is provided with a number of contacting trays represented by broken lines.

In the absorber 10 the feed flows upwardly in countercurrent to solvent, which removes the said acid gases from it. The treated feed leaves from the top of the absorber by a line 14.

Regenerated solvent enters the absorber 10 near its upper end by a line 16 and flows downwardly through the absorber contacting the feed to be treated. Fat solvent leaves from the lower end of the absorber by a line 18 of the solvent circuit leading to a regenerator 20 which the solvent enters at its upper end.

The regenerator 20 shown here comprises a vertical column provided with a number of contacting trays 22 represented by broken lines.

The fat solvent will normally enter the column 3 to 5 trays from the top of the column and will flow downwardly through the trays in a regeneration zone 23 whilst being contacted by rising steam which is generated at the lower end of the column 20 by means of a reboiler 24. The regenerated solvent leaves from the bottom of the regenerator by the line 16 and is returned to the absorber 10 as described above.

In general the pressure of the fat solvent leaving the absorber 10 by line 18 is to be reduced and this can be achieved by a throttle 26, and conversely the regenerated solvent can, if needed, be repressurized before entering the absorber 10.

In addition a heat exchanger 30 enables the fat solvent to be partially heated by the returning hot, regenerated solvent. Further cooling of the regenerated solvent will generally be necessary and is carried out in a cooler 32.

The heat input of the reboiler 24 supplies the heat necessary for the slightly endothermic stripping reaction and the remainder, apart from losses, evaporates a part of the water present in the solvent. The resulting water vapour and the acid gases released during the regeneration leave by the top of the regenerator 20 via a line 34; the water vapour is recondensed in a cooler 36. The acid gases are separated from the resulting condensate in a separator 38 and leave for appropriate further treatment by a line 40.

In general the condensate is recombined with the regenerated solvent in the solvent circuit at 42 downstream of the regenerator via a liquid line 44. Whilst it can be returned to the regenerated solvent between the heat exchanger 30 and the cooler 32 as depicted, the actual place where it is added back in the solvent circuit will largely depend upon its temperature. This will determine whether it is recombined upstream of the heat exchanger 30 or downstream of the cooler 32.

Some of the condensate may be returned to the upper part of the regenerator by a line 46 so as to act as a water wash for the components leaving the regenerator by the line 34. It may also be arranged to heat the cooler, incoming fat solvent entering by the line 18.

Where the solvent is highly concentrated in absorbents, it may alternatively be advantageous to return some or all of the condensate to the reboiler (by a dotted line 50) which will tend to stabilise the conditions in the latter and thus facilitate the control of the regenerator. As a result, the relative partial pressure of the water vapour of the less highly concentrated solution in the reboiler is higher and prevents the temperature from fluctuating wildly as might otherwise occur.

Valve means (not shown) is provided for controlling the proportion of the condensate which is returned to the various points downstream (with respect to the solvent) of the regeneration zone.

Figure 2 represents a plant similar to the one shown in Figure 1 but for carrying out a process according to the invention and also arranged so that a higher proportion (up to all) of the condensate leaving the separator 38 can be recirculated to the upper part 54 of the regenerator 20 by line 46. After descending three tray levels the condensate is trapped by a so-called total draw-off tray 52 which removes all the liquid at that level thus preventing any of the condensate from entering the regeneration zone 23 of the regenerator. This results in a lower steam consumption in the reboiler 24 in comparison with the plant shown in Figure 1 provided condensate is recirculated to the top of the regenerator, in a much better washing of the gases leaving the regenerator and a reduced loss of solvent.

Example and Comparative Experiments
1, 2, and 3

A solvent consisting of 45%w diisopropanolamine (DIPA), 40%w sulfolane (cyclotetramethylenesulphone) and 15%w water, is circulated in a plant as described with reference to Figure 1 of the accompanying drawings.

A natural gas containing 0.5%vol. $H_2S$ as sole acid gas is fed to the absorber and the $H_2S$ content of the treated gas is monitored.

The solvent is circulated at an hourly rate of 100 parts by weight and 10 parts by weight saturated steam are supplied to the reboiler to maintain a regenerator bottom temperature of 140°C.

The regenerator is operated with (Comparative Experiment 1) and without (Comparative Experiment 2) circulation of the condensate to the upper part of the regeneration zone, which amounted to 7 parts by weight per hour. In the case of Comparative Experiment 2 the condensate is cooled and recombined with the regenerated solvent downstream of the regenerator.

The solvent is fed to the absorber at 40°C and the $H_2S$ content of the treated gas is 20 parts by million volume (ppm) in the case where the total condensate is refluxed to the regenerator. The loading of the fat solvent is 0.35 mol $H_2S$/mol amine.

In Comparative Experiment 2, the $H_2S$ content of the treated gas gradually becomes lower. With no change in the steam flow to the regenerator a treated gas containing less than 15 ppm $H_2S$ is obtained.

This indicated the improvement in the stripping in the regenerator when condensate is recombined with regenerated solvent.

Comparative Experiment 1 is repeated with (Comparative Experiment 1) and without (Comparative Experiment 3) reflux of the condensate to the regenerator, but the quantity of steam necessary to achieve a treated gas $H_2S$ content of 20 ppm is measured. As before 10 parts by weight per hour of steam is required to achieve the specification in Comparative Experiment 1, whereas when the condensate is removed and recombined with the lean solvent downstream of the regenerator, after equilibrium was reached, only 7.6 parts by weight of steam per hour is necessary to achieve the specification — a net saving of 24%.

In the event that some of the condensate is circulated into the upper part of the regenerator as a water wash this will increase the steam consumption unless the condensate is withdrawn upstream of the regeneration zone (Example).

## Claims

1. A process for the removal of acid gases from mixtures containing them by contacting the said mixtures in an absorber with a solvent comprising a chemical absorbent and between 5 and 55% by weight of water, regenerating the loaded solvent thus obtained by heating it in a regenerator so that at least part of the water present in the solvent boils and the greater part of the absorbed acid gases is released, recirculating the regenerated solvent to the absorber, withdrawing the resulting water vapour and acid gases from the regenerator, recondensing water vapour from the withdrawn gases, returning condensate thus obtained to the upper part of the regenerator and adding back condensate to the regenerated solvent after it has left the regeneration zone of the regenerator, wherein the regeneration zone is that zone of the regenerator in which solvent is contacted with rising vapour but excludes any reboiling zone at the bottom of the regenerator or exterior thereto, characterized in that condensate is removed from a tray in the regenerator above the regeneration zone.

2. A process as claimed in claim 1, characterized in that at least some of the condensate is added back to the regenerated solvent at the bottom of the regenerator.

3. A process as claimed in claim 1 or 2, characterized in that at least some of the condensate is returned to a reboiler connected to the lower end of the regenerator.

4. A process as claimed in any one of the preceding claims, characterized in that condensate is added back to part of the regenerated solvent and the mixture thus obtained is introduced at the top of the absorber, the remainder of the regenerated solvent being introduced into the absorber at a lower point.

## Patentansprüche

1. Ein Verfahren zum Entfernen saurer Gase aus diese enthaltenden Gemischen durch In-Berührung-Bringen der genannten Gemische in einem Absorber mit einem Lösungsmittel, welches ein chemisches Absorptionsmittel und zwischen 5 und 55 Gewichtsprozent Wasser aufweist, Regenerieren des auf diese Weise erhaltenen beladenen Lösungsmittels durch Erhitzen desselben in einem Regenerator, sodaß zumindest ein Teil des in dem Lösungsmittel vorhandenen Wassers zum Sieden gelangt und der größere Teil der absorbierten sauren Gase freigesetzt wird, Rückführen des regenierten Lösungsmittels in den Absorber, Abziehen des entstandenen Wasserdampfes und der sauren Gase aus dem Regenerator, Rekondensieren von Wasserdampf aus den abgezogenen Gasen, Rückführen von auf diese Weise erhaltenem Kondensat zum oberen Teil des Regerators und Zusetzen von rückgeführtem Kondensat zu dem regenerierten Lösungsmittel, nachdem dieses die Regenerierzone des Regenerators verlassen hat, in dem die Regenerierzone die Zone des Regenerators ist, in welcher Lösungsmittel mit aufsteigendem Dampf in Berührung gebracht wird, nicht jedoch irgendeine am unteren Ende des Regenerators oder außerhalb desselben befindliche Zone zum Wiederverdampfen (reboiler), dadurch gekennzeichnet, daß Kondensat von einem Boden in dem Regenerator oberhalb der Regenerierzone abgezogen wird.

2. Ein Verfahren wie in Anspruch 1 beansprucht, dadurch gekennzeichnet, daß zumindest ein Teil des Kondensats dem regenerierten Lösungsmittel am unteren Ende des Regenerators wieder zugeführt wird.

3. Ein Verfahren wie in Anspruch 1 oder 2 beansprucht, dadurch gekennzeichnet, daß zumindest ein Teil des Kondensats in einen mit dem unteren Ende des Regenerators in Verbindung stehenden Kessel zum Wiederverdampfen rückgeführt wird.

4. Ein Verfahren wie in irgendeinem der vorangehenden Ansprüche beansprucht, dadurch gekennzeichnet, daß Kondensat wieder einem Teil des regenerierten Lösungsmittels zugeführt wird und das auf diese Weise erhaltene Gemisch am oberen Ende des Absorbers eingespeist wird, wobei der Rest des regenerierten Lösungsmittels in den Absorber an einer tiefer gelegen Stelle eingespeist wird.

**Revendications**

1. Un procédé pour l'élimination de gaz acides de mélanges les contenant par mise en contact de ces mélanges dans un absorbeur avec un solvant comprenant un absorbant chimique et de 5 à 55% en poids d'eau, régénération du solvant chargé ainsi obtenu en le chauffant dans un régénérateur de manière qu'au moins une partie de l'eau présente dans le solvant bouille et que la plus grande partie des gaz acides absorbés soit libérée, recyclage du solvant régénéré à l'absorbeur, évacuation du régénérateur de la vapeur d'eau et des gaz acides résultants, recondensation de la vapeur d'eau à partir des gaz évacués, retour du condensat ainsi obtenu à la partie supérieure du régénérateur et addition en retour du condensat au solvant régénéré après qu'il a quitté la zone de régénération du régénérateur, la zone de régénération étant la zone du régénérateur dans laquelle le solvant est mis en contact avec la vapeur qui monte, mais à l'exclusion de toute zone de nouvelle ébullition au fond du régénérateur ou à l'extérieur de lui, caractérisé en ce que le condensat est évacué d'un plateau du régénérateur au-dessus de la zone de régénération.

2. Un procédé selon la revendication 1, caractérisé en ce qu'au moins une partie du condensat est ajoutée en retour au solvant régénéré au fond du régénérateur.

3. Un procédé selon l'une des revendications 1 ou 2, caractérisé en ce qu'au moins une partie du condensat est retournée à un rebouilleur associé à l'extrémité inférieure du régénérateur.

4. Un procédé selon l'une quelconque des revendications précédentes, caractérisé en ce que le condensat est ajouté en retour à une partie du solvant régénéré et le mélange ainsi obtenu est introduit au sommet de l'absorbeur, le reste du solvant régénéré étant introduit dans l'absorbeur à un point situé plus bas.

FIG.1

FIG.2